# EUROPEAN PATENT APPLICATION

(11) **EP 3 632 403 A1**
(43) Date of publication of application: **08.04.2020**
(21) Application number: 18805652.7
(22) Date of filing: 23.05.2018
(51) Int. Cl.: A61K 8/06, A61K 8/14, A61K 8/24

(54) **EMULSIFIED LIPOSOME COMPOSITION AND PREPARATION METHOD THEREFOR**

(30) Priority: 23.05.2017 US 201762510216 P
(71) Applicant: TCI Co., Ltd., Taipei 11494 (TW)
(72) Inventor: LIN, Yung-Hsiang, Taipei City Taiwan 11494 (TW)
(74) Representative: Scholz, Volker
(86) International application number: PCT/CN2018/088051
(87) International publication number: WO 2018/214915

(57) **Abstract**

The present disclosure relates to an emulsified liposome composition and a method for preparing the emulsified liposome composition. The emulsified liposome composition includes a phospholipid, wherein the phospholipid includes a phosphatidyl ethanol amine and a phosphatidylcholine, and a weight ratio of the phosphatidyl ethanol amine to the phosphatidylcholine is 3-6:1. Through the emulsified liposome composition of the present disclosure, the liposome has a small particle diameter, so that it can penetrate into the deep layer of the skin quickly and deeply. Through the moisturizing or anti-oxidant active ingredient encapsulated in the emulsified liposome composition, the emulsified liposome composition has skin moisturizing and anti-oxidation effect, and can be widely used in products regarding maintenance, make-up, and sun-checking.

## Description

### CROSS-REFERENCE TO RELATED APPLICATION

This application claims priority of U.S. patent application No. 62/510,216, filed on May 23, 2017, the content of which is incorporated herein in its entirety by reference.

### BACKGROUND OF THE INVENTION

### 1. Field of the Invention

The present invention relates to a liposome solution, particularly to an emulsified liposome composition and a preparation process thereof.

### 2. The Prior Art

Liposomes are mainly made of phospholipids and are spherical vesicles formed by a lipid bilayer structure including an exposed hydrophilic layer and an internal hydrophobic layer. Since liposomes have a lipid structure similar to that of the cell membrane of organisms, they are well biocompatible and biodegradable, and can be widely used as a carrier for drug administration, release or delivery of related components. For example, the active ingredient is encapsulated in a liposome carrier and administered, which not only has the advantages of increasing the stability of the active ingredient, reducing toxicity and transporting various components, but also reducing the occurrence of allergic conditions, accelerating the metabolism of the body and enhancing the penetration of drugs or active ingredients through the lipid characteristics and size.

In addition to the application to drug delivery, liposomes have been increasingly used in skin care products or cosmetics in recent years. Especially in the skin care products, since the absorption directly affects the function and effect of the active ingredients therein, the characteristics of easy absorption or penetration have always been an important consideration for manufacturers or consumers. In particular, skin care products or cosmetics are applied to the skin, and the skin is the first shielding to foreign substances which cannot be easily penetrated. Therefore, the permeability and penetration rate of the skin to the maintenance-related solutions affect the function of the maintenance ingredients on the skin or deeper tissues. Therefore, through the characteristics of the liposome, the active ingredients encapsulated in the liposome can be passed to the deep layer of the skin, and the maintenance or promotion of the skin care product or the cosmetic can be more effectively exerted. However, the liposome prepared in the general preparation process is too large in particle diameter, which is not conducive to the penetration and absorption of the skin. In addition, because the body is relatively large, the stability of the carrier is insufficient. The component is easily leaked due to rupture of the liposome, and the effect of liposome delivery and function is greatly reduced.

### SUMMARY OF THE INVENTION

A primary objective of the present invention is to provide an emulsified liposome composition which promotes penetration and absorption of the encapsulated active ingredient through liposome having a smaller particle diameter in the emulsified liposome solution.

Another objective of the present invention is to provide an emulsified liposome composition having excellent moisturizing effect and antioxidant activity, which enhances the skin moisturizing ability and antioxidant capacity by combining the active ingredients of moisturizing and anti-oxidizing effects with the penetration of liposomes.

Another objective of the present invention is to provide a liposome composition having high temperature sterilization treatment characteristics, wherein the liposome can maintain a stable structure after high temperature treatment, so as to be applicable to various products being treated with the pretreatment of sterilization process.

Another objective of the present invention is to provide a method for preparing an emulsified liposome composition which is subjected to emulsification and homogenization at a high pressure and an appropriate temperature to prepare the emulsified liposome composition containing a liposome having a small particle diameter.

According to an embodiment of the present invention, the emulsified liposome composition comprises a phospholipid, wherein the phospholipid comprises a phosphatidyl ethanol amine and a phosphatidylcholine, and a weight ratio of the phosphatidyl ethanol amine to the phosphatidylcholine is 3-6:1, preferably 4:1.

According to an embodiment of the present invention, the emulsified liposome composition forms a liposome, wherein the liposome maintains an intact structure after being treated at 95 °C for 30 minutes.

According to an embodiment of the present invention, the phospholipid is in an amount of 0.1% by weight to 2% by weight based on 100% by weight of the emulsified liposome composition.

According to an embodiment of the present invention, the emulsified liposome composition comprises a first component group consisting of 3% to 7% by weight of Butane-1,3-diol, 0.4% to 0.7% by weight of hydroxyacetophenone, and 0.4% to 0.7% by weight of Hexane-1,6-diol, a second component group consisting of 1% to 5% by weight of glycerol, 0.01% to 0.3% by weight of Xanthan Gum, and 0.1% to 0.3% by weight of Hydrolyzed sclerotium gum, a third component group consisting of 0.5% to 10% by weight of an active ingredient and 0.1% to 2% by weight of lecithin, and water.

According to an embodiment of the present invention, the active ingredient in the emulsified liposome composition can be selected from the group consisting of red quinoa extract, coconut oil, anti-allergic plant complex, saccharide isomerate, glycosyl trehalose, and combinations thereof.

According to an embodiment of the present invention, the active ingredient consists of 0.1% to 2% by weight of red quinoa extract, 0.1% to 2% by weight of coconut oil, 0.1% to 2% by weight of anti-allergic plant complex, 0.1% to 2% by weight of saccharide isomerate, and 0.1% to 2% by weight of glycosyl trehalose.

According to an embodiment of the present invention, the liposome formed in the emulsified liposome composition has a particle diameter of 10 nm to 1000 nm, preferably 300 nm to 400 nm, more preferably about 350 nm.

According to an embodiment of the present invention, the present invention provides a method for preparing an emulsified liposome composition, comprising the following steps: preparing a first solution, including providing 3% to 7% by weight of Butane-1,3-diol, 0.4% to 0.7% by weight of hydroxyacetophenone, and 0.4% to 0.7% by weight of Hexane-1,6-diol, mixing evenly and dispersing, heating at 60 °C to 80 °C until the solution becomes transparent and no undissolved particles, and cooling to 25 °C to 30 °C; preparing a second solution, including providing 1% to 5% by weight of glycerol, 0.01% to 0.3% by weight of Xanthan Gum, and 0.1% to 0.3% by weight of Hydrolyzed sclerotium gum, mixing evenly and dispersing, adding 74%-94.4% by weight of water, heating at 60 °C to 80 °C until the glycerol, the Xanthan Gum and the Hydrolyzed sclerotium gum have no agglomeration, and cooling to 25 °C to 30 °C; preparing a third solution, including providing 0.5% to 10% by weight of an active ingredient and 0.1% to 2% by weight of lecithin, and adding the active ingredient and the lecithin to the second solution, followed by stirring evenly at 1500 rpm to 3500 rpm for 5 to 15 minutes; preparing a fourth solution, including homogenizing the third solution at a pressure of 300 bar to 600 bar and a temperature of 25 °C to 30 °C; and adding the fourth solution to the first solution, followed by stirring evenly at 1500 rpm to 3500 rpm for 5 to 15 minutes.

According to an embodiment of the present invention, the active ingredient in the emulsified liposome composition prepared by the above preparation method can be selected from the group consisting of red quinoa extract, coconut oil, anti-allergic plant complex, saccharide isomerate, glycosyl trehalose, and combinations thereof.

According to an embodiment of the present invention, the active ingredient in the emulsified liposome composition prepared by the above preparation method consists of 0.1% to 2% by weight of red quinoa extract, 0.1% to 2% by weight of coconut oil, 0.1% to 2% by weight of anti-allergic plant complex, 0.1% to 2% by weight of saccharide isomerate, and 0.1% to 2% by weight of glycosyl trehalose.

According to an embodiment of the present invention, the liposome formed in the emulsified liposome composition prepared by the above preparation method has a particle diameter of 10 nm to 1000 nm, preferably 300 nm to 400 nm, more preferably about 350 nm.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.
FIG. 1 is a diagram showing the comparison result of particle diameter distribution analysis of a liposome solution prepared in the example of the present invention, wherein (A) is the emulsified liposome composition prepared in Example 1, and (B) is the general liposome solution prepared in Example 2.
FIG. 2 is an image of a liposome observed by an atomic force microscope of the emulsified liposome composition prepared in Example 1 of the present invention.
FIG. 3 is a diagram showing the comparison result of liposome solutions prepared in Example 1 (test group) of the present invention and Example 2 (control group) in the skin moisturizing test of the arm.
FIG. 4 is a diagram showing the comparison result of the liposome structural stability test of the liposome solution prepared in Example 1 (test group) of the present invention and the liposome solution prepared by different lecithin (comparative group).
FIG. 5 is an analysis diagram showing the components (A and B) contained in the lecithin used in Example 1 (test group) of the present invention and the components contained in the lecithin used in the test group (C) and the comparative group (D).
FIG. 6 is a diagram showing the comparison result of liposome solutions prepared in Example 1 (test group) of the present invention and Example 2 (control group) in the skin moisturizing test of the face.
FIG. 7 is a diagram showing the comparison result of liposome solutions prepared in Example 1 (test group) of the present invention and Example 2 (control group) in the melanin index.
FIG. 8 is a diagram showing the comparison result of the effect of ultraviolet spot number on liposome solutions prepared in Example 1 (test group) of the present invention and Example 2 (control group).
FIG. 9 is a diagram showing the comparison result of the effects of liposome solutions prepared in Example 1 (test group) of the present invention and Example 2 (control group) on the degree of skin redness.
FIG. 10 is a diagram showing the comparison result of liposome solutions prepared in Example 1 (test group) of the present invention and Example 2 (control group) in the effect of wrinkle reduction ratio.

### DETAILED DESCRIPTION OF THE PREFERRED EMBODIMENT

In the following detailed description of the embodiments of the present invention, reference is made to the accompanying drawings, which are shown to illustrate the specific embodiments in which the present disclosure may be practiced. These embodiments are provided to enable those skilled in the art to practice the present disclosure. It is understood that other embodiments may be used and that changes can be made to the embodiments without departing from the scope of the present invention. The following description is therefore not to be considered as limiting the scope of the present invention.

As used herein, the data provided represent experimental values that can vary within a range of ±20%, preferably within ±10%, and most preferably within ±5%.

First, an appropriate amount of the components required for preparing the emulsified liposome composition was prepared, wherein hydroxyacetophenone (trade name: SymSave) was purchased from Philicia Inc.; xanthan gum (trade name: Rhodicare T) was purchased from TOP RHYME INTERNATIONAL CO., LTD.; hydrolyzed sclerotium gum (trade name: BioNest-Chcogum HG) was purchased from HonorChem Co., Ltd.; red quinoa extract was purchased from TCI Co., Ltd.; coconut oil was purchased from KLIGATE TW CO., LTD.; anti-allergic plant complex (trade name: BIOPHYTEXTM LS 9832) was purchased from P-MORE Co., Ltd.; saccharide isomerate was purchased from ESSENCE PLUS CO., LTD.; glycosyl trehalose (trade name: Tornare) was purchased from KALIN ENTERPRISE CO., LTD.; and lecithin (trade name: SOLEC) was purchased from Solae, LLC, USA, which may be soy lecithin and the like. The foregoing components are not limited to the source of the purchase, and the same or similar components may be used. The remaining compounds or solutions for preparation are also not limited to the source of the purchase, as long as the components are the same.

### Example 1

### Preparation of emulsified liposome composition

Referring to the components listed in Table 1, each component of a first component group consisting of 3% to 7% by weight of Butane-1,3-diol, 0.4% to 0.7% by weight of hydroxyacetophenone and 0.4% to 0.7% by weight of Hexane-1,6-diol was prepared in an amount of 100% by weight of the emulsified liposome composition. After mixing evenly and dispersing, heating was carried out at 60 °C to 80 °C until the solution becomes transparent and there are no undissolved particles, and the temperature was lowered to 25 °C to 30 °C for use. The solution thus prepared is called the first solution. Further, each component of a second component group consisting of 1% to 5% by weight of glycerol, 0.01% to 0.3% by weight of xanthan gum, and 0.1% to 0.3% by weight of Hydrolyzed sclerotium gum was prepared. Firstly, each component of the second component group was mixed evenly and dispersed, and 74% to 94.4% by weight of deionized water was added, followed by heating at 60 °C to 80 °C until the glycerol, the Xanthan Gum and the Hydrolyzed sclerotium gum have no agglomeration. The temperature was lowered to 25 °C to 30 °C for use. The solution thus prepared is called the second solution. Each component of a third component group consisting of 0.1% to 2% by weight of red quinoa extract, 0.1% to 2% by weight of coconut oil, 0.1% to 2% by weight of anti-allergic plant complex, 0.1% to 2% by weight of saccharide isomerate, 0.1% to 2% by weight of glycosyl trehalose, and 0.1% to 2% by weight of lecithin was sequentially added to the second solution, followed by stirring at 1500 rpm-3500 rpm for 5 minutes to 15 minutes until uniform. The solution thus prepared is called the third solution. Thereafter, the third solution was homogenized by a high pressure homogenizer at a pressure of 300 bar to 600 bar and a temperature of 25 to 30 °C, and the solution thus treated is referred to as the fourth solution. Finally, the fourth solution was added to the first solution prepared as described above, followed by stirring at a rotational speed of 1500 rpm to 3500 rpm for 5 minutes to 15 minutes to complete the preparation of the emulsified liposome composition.

**Table 1**

| Component group | component | Weight ratio (%) |
|---|---|---|
| - | water | 74-94.4 |
| First component group | Butane-1,3-diol | 3-7 |
| | hydroxyacetophenone | 0.4-0.7 |
| | Hexane-1,6-diol | 0.4-0.7 |
| Second component group | glycerol | 1-5 |
| | xanthan gum | 0.1-0.3 |
| | Hydrolyzed sclerotium gum | 0.1-0.3 |
| Third component group | red quinoa extract | 0.1-2 |
| | coconut oil | 0.1-2 |
| | anti-allergic plant complex | 0.1-2 |
| | saccharide isomerate | 0.1-2 |
| | glycosyl trehalose | 0.1-2 |
| | lecithin | 0.1-2 |

### Example 2

### Preparation of general essence solution

In this example, the source of the components required for preparing the general essence solution may be the same as in Example 1. Referring to the components listed in Table 2, each component of the first component group consisting of 3% to 7% by weight of Butane-1,3-diol, 0.4% to 0.7% by weight of hydroxyacetophenone and 0.4% to 0.7% by weight of Hexane-1,6-diol was prepared in an amount of 100% by weight of the liposome composition. After mixing evenly and dispersing, heating was carried out at 60 °C to 80 °C until the solution becomes transparent and there are no undissolved particles, and the temperature was lowered to 25 °C to 30 °C for use. The solution thus prepared is called the first solution. Further, each component of the second component group consisting of 1% to 5% by weight of glycerol, 0.01% to 0.3% by weight of xanthan gum, and 0.1% to 0.3% by weight of Hydrolyzed sclerotium gum was prepared. Firstly, each component of the second component group was mixed evenly and dispersed, and 76% to 94.5% by weight of deionized water was added, followed by heating at 60 °C to 80 °C until the glycerol, the Xanthan Gum and the Hydrolyzed sclerotium gum have no agglomeration. The temperature was lowered to 25 °C to 30 °C for use. The solution thus prepared is called the second solution. Each component of a comparative third component group consisting of 0.1% to 2% by weight of red quinoa extract, 0.1% to 2% by weight of coconut oil, 0.1% to 2% by weight of anti-allergic plant complex, 0.1% to 2% by weight of saccharide isomerate, and 0.1% to 2% by weight of glycosyl trehalose was sequentially added to the second solution, followed by stirring at 1500 rpm-3500 rpm for 5 minutes to 15 minutes until uniform. The solution thus prepared is called the comparative third solution. The comparative third solution was added to the first solution prepared as described above, followed by stirring at a rotational speed of 1500 rpm to 3500 rpm for 5 minutes to 15 minutes to complete the preparation of the general liposome composition.

**Table 2**

| Component group | component | Weight ratio (%) |
|---|---|---|
| - | water | 76-94.5 |
| First component group | Butane-1,3-diol | 3-7 |
| | hydroxyacetophenone | 0.4-0.7 |
| | Hexane-1,6-diol | 0.4-0.7 |
| Second component group | glycerol | 1-5 |
| | xanthan gum | 0.1-0.3 |
| | Hydrolyzed sclerotium gum | 0.1-0.3 |
| Third component group | red quinoa extract | 0.1-2 |
| | coconut oil | 0.1-2 |
| | anti-allergic plant complex | 0.1-2 |
| | saccharide isomerate | 0.1-2 |
| | glycosyl trehalose | 0.1-2 |

### Example 3

### Analysis of particle diameter distribution of liposomes in liposome composition

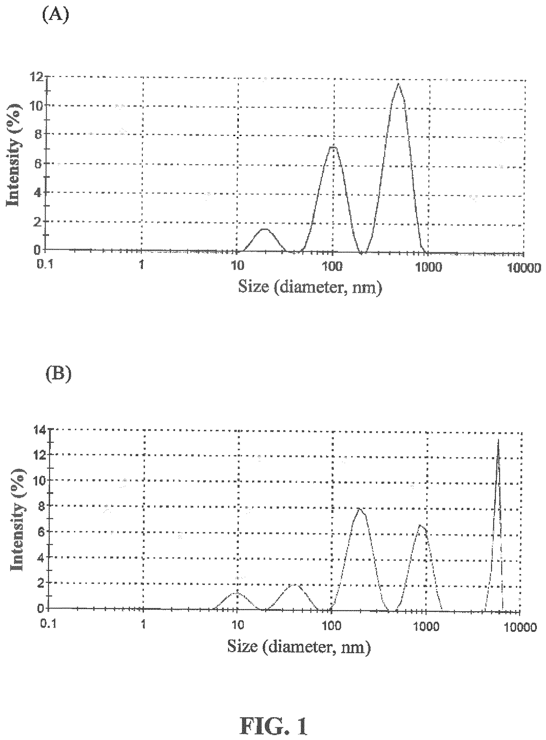

The emulsified liposome composition prepared in Example 1 was used as a test group, the general liposome composition prepared in Example 2 was used as a control group, and the following particle diameter distribution analysis was performed. First, 2 ml of the emulsified liposome composition prepared in Example 1 was taken and diluted with 1:1 (v/v) deionized water. Similarly, 2 ml of the liposome composition prepared in Example 2 was taken and diluted with 1:1 (v/v) deionized water. Then, 2 ml of the diluted solution was taken out separately for particle diameter analysis by Dynamic Light Scattering (DLS), and the result of the particle diameter distribution was shown by the detection intensity, as shown in FIG. 1.

From the result of (A) in FIG 1, the emulsified liposome composition (test group) prepared by the special component and the preparation method of the present invention has a particle diameter ranging from 100 nm to 1000 nm. There are more distributions between 40 to 200 nm and 200 to 1000 nm, and the average particle diameter is 350 nm. The liposome solution (control group) obtained by the general preparation method of FIG. 1 (B) has an average particle diameter of 4716 nm, and the particle diameter is obviously much larger than that of the test group. Therefore, the emulsified liposome composition prepared by the components and the preparation method in the examples of the present invention can effectively promote the penetration and absorption of the skin because of having a small particle diameter distribution.

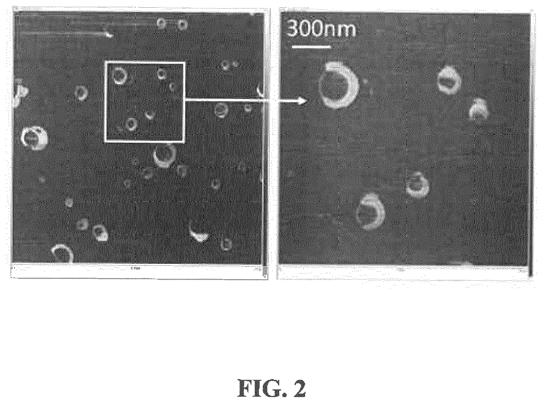

Further, the emulsified liposome composition prepared in Example 1 of the present invention was placed on a mica sheet, and observed by an atomic force microscope (Atomic Force Microscope System with ScanAsyst, Dimension Icon). The result is shown in FIG. 2. From the result of FIG. 2, it was found that the emulsified liposome composition prepared by the present invention allows the liposome to have a bilayer structure. Basically, the components of the third component group are encapsulated on the bilayer membrane formed by the liposome, and components of the first component group and the second component group, and few components of the third component group are encapsulated in the core cavity of the liposome.

### Example 4

### Transdermal absorption test of emulsified liposome composition

The liposome solutions prepared in Example 1 and Example 2 were also prepared as the test group and the control group, respectively. An appropriate amount of the liposome composition was evenly mixed with 0.1 µg-1 µg of Dil fluorescent dye (DiTC₁₈(3)), and then applied to the treated pig ear skin at 0 minute, 5 minutes, 15 minutes and 8 hours after application, respectively. Thereafter, the result was observed and photographed using a confocal microscope.

It can be seen from the result that the test group can be observed a significant red fluorescence infiltration under the skin within 5 minutes, and after 15 minutes of application, the penetration depth has reached about 200 µm. In contrast, the control group showed a penetration depth of only about 30 µm after 15 minutes of application, showing that the emulsified liposome composition prepared by the present invention has excellent penetration and can quickly penetrate into the skin.

### Example 5

### Skin moisturizing test of arm using emulsified liposome composition

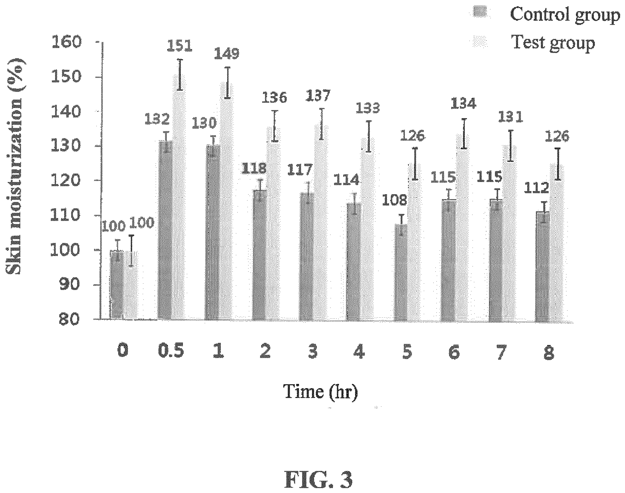

The liposome compositions prepared in Example 1 and Example 2 were also prepared as the test group and the control group, respectively. An appropriate amount of the liposome composition was applied to the inner side of the test subject's arm. At 0 hour, 0.5 hours, 1 hour, 2 hours, 3 hours, 4 hours, 5 hours, 6 hours, 7 hours and 8 hours after administration, the skin moisture content measurement probe (Courage+Khazaka electronic GmbH) was used to measure the moisture content of the skin, and the result is shown in FIG. 3. From the result of FIG. 3, when the emulsified liposome composition of the test group was applied, the average skin water content was about 14% to 20% higher than that of the control group at any measurement time point. After 8 hours, the average skin moisture content of the test group was still 26% higher than that before use, showing that the emulsified liposome composition prepared by the present invention not only has excellent moisturizing effect, but also has long-lasting moisturizing properties.

### Example 6

### Structural integrity testing of liposomes in liposome composition

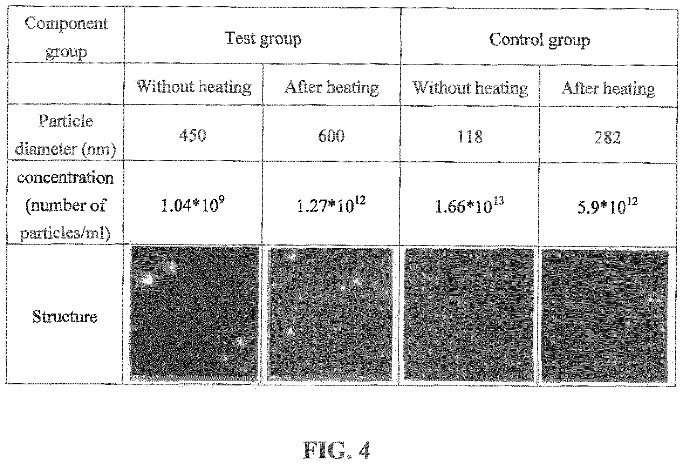

The liposome composition prepared in Example 1 was prepared as the test group, and the liposome composition prepared by the same components and methods as in Example 1 was used as the comparative group, except that lecithin was replaced with other commercially available lecithin (EMULMETIK 900, Lucas Neyer Cosmetics). The test group and the comparative group were respectively heated at 95 °C for 30 minutes under heating conditions equivalent to sterilization, the particle diameter and concentration were measured, and the structure of the liposome was observed. The result is shown in FIG. 4. As shown in FIG. 4, the structure of the emulsified liposome composition prepared by the present invention was still intact after heating at a high temperature, that is, its structural stability was higher than that of the comparative group. The emulsified liposome composition having high temperature resistance will be used in products that must be sterilized, which would greatly improve the stability of the product.

### Example 7

### Differences in lecithin in liposome composition

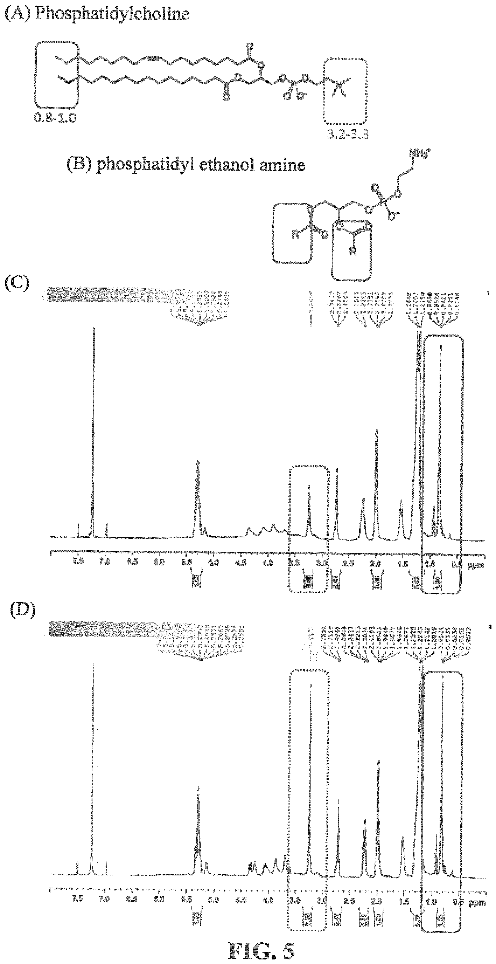

The lecithin used in Example 1 and the lecithin used in Example 6 were subjected to NMR spectral alignment, and the results are shown in (C) and (D) of FIG. 5, respectively. (A) and (B) in FIG. 5 are the chemical structural formulas of phosphatidylcholine and phosphatidyl ethanol amine contained in lecithin, respectively. As shown in (C), the ratio of phosphatidyl ethanol amine to phosphatidylcholine contained in the lecithin used in the test group of Example 1 is about 4:1. FIG. 5 (D) shows that the ratio of the comparative group is about 1:1. It is speculated that it may be because the phosphatidylcholine has more amine groups, resulting in instability of the liposome structure in the comparative group.

### Example 8

### Facial clinical testing of liposome composition

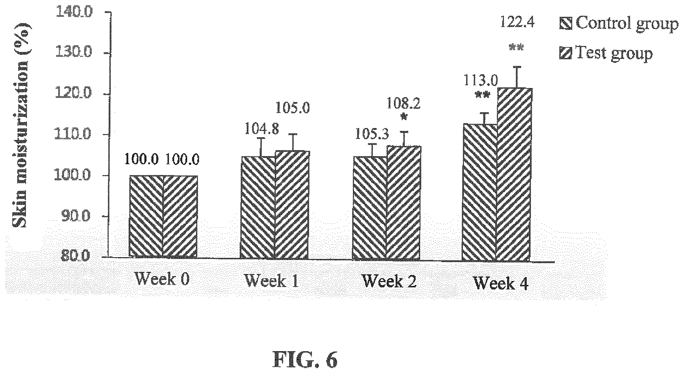

The liposome compositions prepared in Example 1 and Example 2 were prepared as the test group and the control group after appropriate dilution. Thereafter, the mask which was dried without adding any ingredients was cut into two left and right pieces for use. One of them was soaked in a dilution of 10 ml of the test group, and the other was soaked with a dilution of 10 ml of the control group. Thereafter, the left and right masks were applied to the left and right faces of 10 subjects for about 15 minutes. Applying 3 times a week, and the following skin moisturizing, melanin index, ultraviolet spot number, skin redness and wrinkle reduction ratio on the 0th, 1st, 2nd, and 4th week were observed or tested, respectively. The results are shown in FIGs 6 to 10. Regarding the moisturizing test of the facial skin, the test method of Example 5 can be utilized. The melanin index can be detected by a melanin detection probe (Courage+Khazaka electronic GmbH), and the rest is quantified by image statistics.

Referring to FIG. 6, FIG. 6 is a diagram showing the comparison result of liposome compositions prepared in Example 1 (test group) of the present invention and Example 2 (control group) in the skin moisturizing test of the face. From the result of FIG. 6, it was found that when the emulsified liposome composition of the test group was administered, the moisture content of the facial skin was about 22.4% higher than that of the control group after the fourth week of administration, showing that the emulsified liposome composition prepared by the present invention not only has an excellent moisturizing effect on the arm but also on the face.

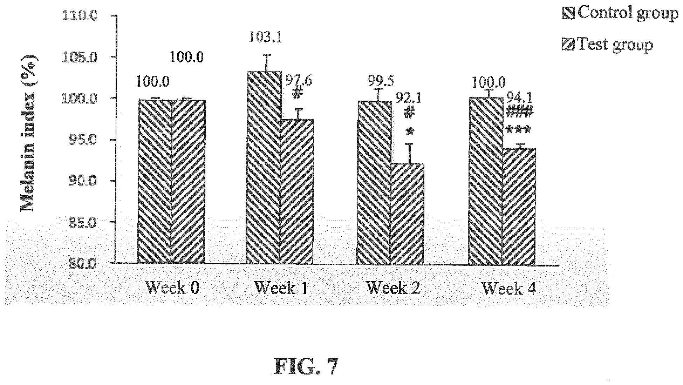

Referring to FIG. 7, FIG. 7 is a diagram showing the comparison result of liposome compositions prepared in Example 1 (test group) of the present invention and Example 2 (control group) in the melanin index. From the result of FIG. 7, it was found that when the emulsified liposome composition of the test group was administered, the melanin index was significantly reduced by 5.9% after the fourth week of administration, showing that the emulsified liposome composition prepared by the present invention has excellent whitening effect.

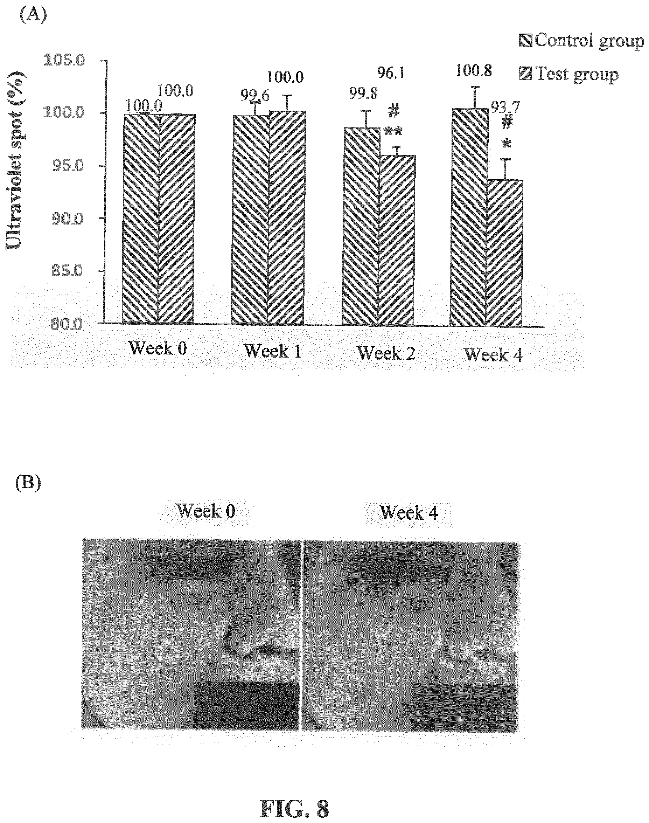

Referring to FIG. 8, FIG. 8 is a diagram showing the comparison result of the effect of ultraviolet spot number on liposome compositions prepared in Example 1 (test group) of the present invention and Example 2 (control group). From the result of FIG. 8, it was found that when the emulsified liposome composition of the test group was applied, the ultraviolet spot numbers were also significantly reduced by 6.3% after the fourth week of application, showing that the emulsified liposome composition prepared by the present invention has good effect on skin repair after sunburn.

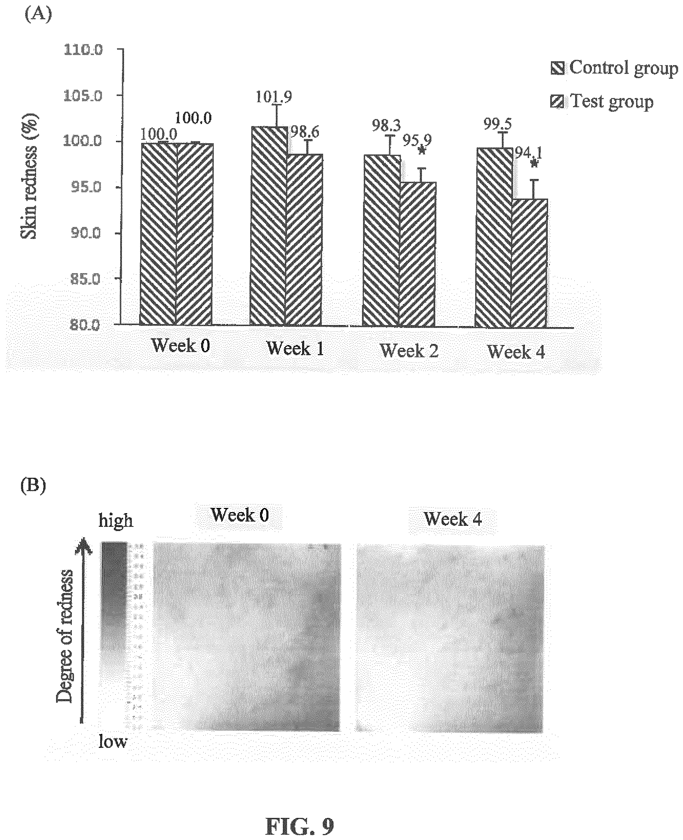

Referring to FIG. 9, FIG. 9 is a diagram showing the comparison result of the effects of liposome compositions prepared in Example 1 (test group) of the present invention and Example 2 (control group) on the degree of skin redness. As can be seen from the result of FIG. 9, when the emulsified liposome composition of the test group was administered, the redness and inflammation of the skin was significantly reduced by 5.9% after the fourth week of administration, showing that the emulsified liposome composition prepared by the present invention has good effect on anti-skin inflammation.

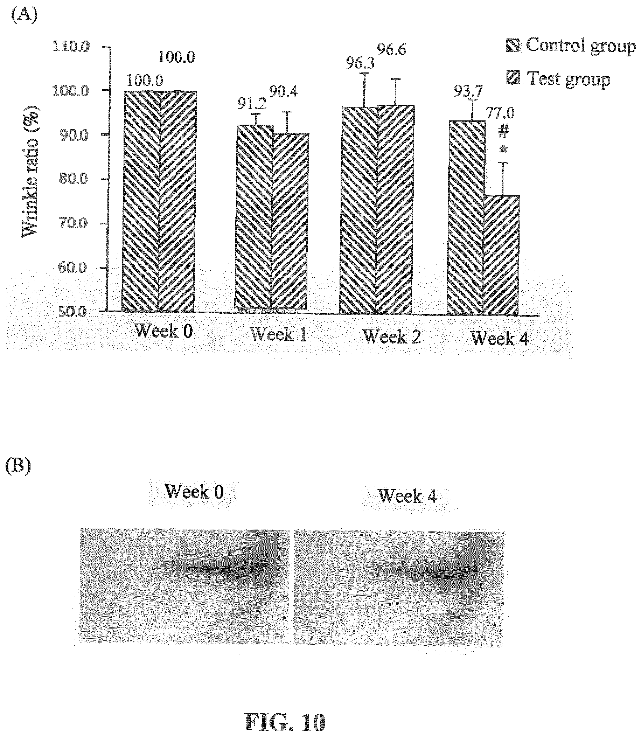

Referring to FIG. 10, FIG. 10 is a diagram showing the comparison result of liposome compositions prepared in Example 1 (test group) of the present invention and Example 2 (control group) in the effect of wrinkle reduction ratio. As can be seen from the result of FIG. 10, when the emulsified liposome composition of the test group was applied, the reduction of wrinkles was by up to 23% after the fourth week of administration, showing that the emulsified liposome composition prepared by the present invention has excellent effect on smoothing skin wrinkles.

According to the above test, the emulsified liposome composition of the embodiment of the present invention can effectively exert the effect by rapidly transmitting the encapsulated active ingredient to the deep layer of the skin due to the small particle diameter, the better penetration force and the permeation rate. Therefore, the effects of the active ingredients can be effectively exerted.

Therefore, the emulsified liposome composition of the embodiment of the present invention can be further applied to moisturizers, skin moisturizers, essences, lotions, make-up removers, aftershaves, mascara or eyeliner, barrier creams, and even shampoo and conditioner, as well as sun care products, anti-aging or anti-wrinkle, whitening and other care products.

Although the present invention has been described with reference to the preferred embodiments, it will be apparent to those skilled in the art that a variety of modifications and changes in form and detail may be made without departing from the scope of the present invention defined by the appended claims.

## Claims

1. An emulsified liposome composition, comprising a phospholipid, wherein the phospholipid comprises a phosphatidyl ethanol amine and a phosphatidylcholine, and a weight ratio of the phosphatidyl ethanol amine to the phosphatidylcholine is 3-6:1.

2. The emulsified liposome composition according to claim 1, wherein the weight ratio of the phosphatidyl ethanol amine to the phosphatidylcholine is 4:1.

3. The emulsified liposome composition according to claim 1, which forms a liposome, wherein the liposome maintains an intact structure after being treated at 95 °C for 30 minutes.

4. The emulsified liposome composition according to claim 1, which forms a liposome, wherein the liposome has a particle diameter of 10 nm to 1000 nm.

5. The emulsified liposome composition according to claim 4, wherein the particle diameter of the liposome ranges from 300 nm to 400 nm.

6. The emulsified liposome composition according to claim 1, wherein the phospholipid is in an amount of 0.1% by weight to 2% by weight based on 100% by weight of the emulsified liposome composition.

7. A method for preparing an emulsified liposome composition, comprising the following steps:
preparing a first solution, including providing 3% to 7% by weight of Butane-1,3-diol, 0.4% to 0.7% by weight of hydroxyacetophenone, and 0.4% to 0.7% by weight of Hexane-1,6-diol, followed by mixing evenly and dispersing;
preparing a second solution, including providing 1% to 5% by weight of glycerol, 0.01% to 0.3% by weight of Xanthan Gum, and 0.1% to 0.3% by weight of Hydrolyzed sclerotium gum, mixing evenly and dispersing, adding 74%-94.4% by weight of water, heating until the glycerol, the Xanthan Gum and the Hydrolyzed sclerotium gum have no agglomeration, and cooling down;
preparing a third solution, including providing 0.5% to 10% by weight of an active ingredient and 0.1% to 2% by weight of lecithin, and adding the active ingredient and the lecithin to the second solution, followed by stirring evenly;
preparing a fourth solution, including homogenizing the third solution at a pressure of 300 bar to 600 bar and a temperature of 25 °C to 30 °C; and
adding the fourth solution to the first solution, followed by stirring evenly.

8. The method according to claim 7, wherein the lecithin comprises a phospholipid, the phospholipid comprises a phosphatidyl ethanol amine and a phosphatidylcholine, and a weight ratio of the phosphatidyl ethanol amine to the phosphatidylcholine is 3-6:1.

9. The method according to claim 7, wherein the emulsified liposome composition forms a liposome, and the liposome has a particle diameter of 10 nm to 1000 nm.

10. The method according to claim 9, wherein the particle diameter of the liposome ranges from 300 nm to 400 nm.
